# EUROPEAN PATENT APPLICATION

(11) **EP 3 735 959 A1**
(43) Date of publication of application: **11.11.2020**
(21) Application number: 19382347.3
(22) Date of filing: 07.05.2019
(51) Int. Cl.: A61K 8/34, A61Q 19/02, A61K 8/9783, A61K 8/9767

(54) **COMPOSITIONS TO REDUCE HYPERPIGMENTATION**

(71) Applicant: InnovativeHealth Group SL, 28049 Madrid (ES)
(72) Inventor: Millán Ortega, Estrella, 28822 Madrid (ES); García González, Víctor, 28830 Madrid (ES); Mellado Fuentes, Ana María, 14001 Córdoba (ES); Collado Rojas, Juan Antonio, 14004 Córdoba (ES); Muñoz Molina, Eduardo, 28049 Madrid (ES); Muñoz Blanco, Eduardo, 14004 Córdoba (ES)
(74) Representative: Evonik Patent Association

(57) **Abstract**

A composition comprising cannabidiol, *Coriandrum sativum* and *Pinus radiata* bark extracts, as wells as to cosmetic and pharmaceutical formulations comprising said composition, are described. Additionally described is the use of said composition and said cosmetic formulations in non-therapeutic cosmetic methods, to reduce and/or prevent the appearance of skin hyperpigmentation, as well as the use of said composition and said pharmaceutical formulations as a medicament, in particular in the prevention and/or treatment of hyperpigmentation disorders.

## Description

### Technical field

The present invention refers to a composition comprising cannabidiol, *Coriandrum sativum* and *Pinus radiata* bark extracts, as wells as to cosmetic and pharmaceutical formulations comprising said composition. Additionally, present invention refers to the use of said composition and cosmetic formulations containing the same in non-therapeutic cosmetic methods for reducing and/or preventing the appearance of skin hyperpigmentation, as well as to the use of said composition and pharmaceutical formulations containing the same as a medicament, in particular in the prevention and/or treatment of hyperpigmentation disorders.

### Background art

Human skin is a physical barrier that performs several essential functions in the organism. Its main role consists on ensuring the protection of our body against a variety of damaging stimuli such as ultraviolet radiation (UV), environmental pollution, mechanical injury or different chemicals¹.

Skin pigmentation is an important process that participates in the defense against UV radiation. This process, known as melanogenesis, is due to the accumulation of melanin pigments in skin cells. Constitutive skin pigmentation reflects the genetically determined level of synthesized melanin and can be modulated by several intrinsic and extrinsic factor, leading to hyperpigmentation or hypopigmentation of skin¹.

Principally, there are two types of epidermal cells involved in skin pigmentation, keratinocytes, and melanocytes. The relationship between these cells is important in the regulation of the skin pigmentation process¹.

In the epidermis, melanocytes, which are responsible for the production of melanin pigments, are surrounded by keratinocytes. Melanocytes contain melanosomes, subcellular lysosome-like organelles in which melanin pigments are synthesized and stored before its distribution to the surrounding keratinocytes. Melanosomes require several specific enzymatic and structural proteins to produce melanin. Some of them are tyrosinase (TYR), tyrosinase-related protein 1 (TYRP1 or TRP1), tyrosinase-related protein 2 (TYRP2 or TRP2) and microphthalmia-associated transcription factor (MITF)².

There are two main types of synthesized melanin pigments, pheomelanin (reddish yellow) and eumelanin (brownish black), and, their combination leads to the skin coloring¹. Melanin production starts in the cytosol when L-phenylalanine is converted to L-tyrosine by phenylalanine hydroxylase (PAH)². Pheomelanin and eumelanin derive from a single precursor, the amino acid L-tyrosine¹, and the synthesis of pheomelanin and eumelanin involves the activation of the key enzyme of melanogenesis, tyrosinase. This enzyme catalyzes the first two steps of melanin production: the hydroxylation of L-tyrosine to L-dihydroxyphenylalanine (L-DOPA) and the subsequent oxidation of L-DOPA to the corresponding quinone, L-DOPAquinone³.

Following the formation of DOPAquinone, the melanin pathway is divided into the synthesis of the black-brownish eumelanin and synthesis of the red-yellow pheomelanin³. In the eumelanin pathway, the absence of thiol compounds spontaneously transforms L-DOPAquinone, following intramolecular cyclization, into DOPAchrome via leuko-DOPAchrome) intermediate¹. Then, DOPAchrome is converted to 5,6-dihydroxyindole-2-carboxylic acid (DHICA) via enzymatic conversion by DOPAchrome tautomerase, also known as tyrosine-related protein-2 (TYRP-2)³. In parallel, DOPAchrome may undergo decarboxylation by spontaneous tautomerization into 5,6-dihydroxyindole (DHI). The ratio of DHICA/DHI units depends on TYRP2 activity and on the spontaneous decarboxylation rate. Further oxidation of DHI by tyrosinase or TYRP1 generates the corresponding indolequinones, IQ (5,6-indolequinone) and IQCA (indole-5,6-quinone carboxylic acid). The reduced forms (hydroxyindoles) as DHI and the oxidized ones (indolequinones) as IQ or IQCA are finally assembled by cross-linking reactions into eumelanin pigment¹. The pheomelanin pathway branches from the eumelanin pathway at the L-DOPAquinone step and is dependent on the presence of L-cysteine. Cysteine reacts with L-DOPAquinone³ to produce several cysteinyl-DOPA (CD) isomers, such as cysteinyl-DOPA. Further oxidation of these aforesaid isomers and their polymerization lead to the formation of pheomelanin via benzothiazine intermediates¹.

Redox conditions are crucial for the balance between the production of eumelanin and pheomelanin. The formation of eumelanin or pheomelanin is directly determined by reduced glutathione (GSH), wherein high levels of GSH are related with the eumelanin synthesis process, and low GSH concentrations are associated with the synthesis of the pheomelanin pigment³. Therefore, it has been proposed that melanocytes can act as regulators for local and global homeostasis of melanogenic systems by controlling L-tyrosine levels and L-DOPA production².

The signaling pathways involved in melanogenesis converge mainly at MITF, the microphthalmia-associated transcription factor¹. MITF plays a central role in a regulatory network of transcription factors and signaling pathways that control the survival, proliferation, and differentiation of melanocytes. Not only the melanocyte development is affected by this protein but also the pigmentation via its transcriptional regulatory effect on tyrosinase, TRP-1, and TRP-2. Therefore, MITF plays a central role in melanin synthesis, as well as melanosome biogenesis and transport³.

Reactive oxygen species (ROS) are produced by mitochondria and peroxisomes during normal cellular metabolic processes. The ROS production may be increased upon exposure to exogenous factors, such as ultraviolet radiation (UV). Epidermal melanocytes are particularly vulnerable to an excess of reactive oxygen species production due to their specialized function: melanin synthesis, which is stimulated by sun exposure that results in hyperpigmentation of skin⁴.

As discussed earlier, skin pigmentation can be modified by many internal and external events. In fact, during pregnancy, the female can develop melasma, a skin condition produced by the hormonal changes that cause dark patches on both sides of the face. Additionally, the aging process produces dark spots in the skin, known as solar lentigines. Apart from this internal events, other external factors can alter the skin pigmentation process, as the topical treatment with different active compounds⁵.

Given this context, many cosmetic and pharmaceutical companies are investigating what factors can alter skin pigmentation. Nowadays, there are many known substances that can reduce the level of pigmentation in the skin. Many of these substances have a tyrosinase-inhibiting effect that leads to reduced total melanin production. Some of the tyrosinase inhibitors used today are for example kojic acid, arbutin and different kinds of vegetal or herb extracts³.

Cannabidiol (CBD) is a phytocannabinoid extracted from the plant *Cannabis sativa.* CBD displays anti-inflammatory, antioxidant and anti-apoptotic effects in many different cell types including skin cells. A report has shown that CBD at high concentration (6 µM) induced melanogenesis by increasing the expression of MITF by activating p38 MAPK and p42/44 MAPK⁶. In addition, cannabidiol treatment in human epidermal melanocytes significantly increased cellular tyrosinase activity in a concentration dependent manner. These results are indicative that cannabidiol can induce melanogenesis in human melanocytes⁶. In the same trend, another study demonstrated that non-cytotoxic doses of endocannabinoids (eCBs) can enhance melanin synthesis through a CB₁ receptor-dependent activation of tyrosinase gene expression mediated by p38 and p42/44 MAPKs, CREB, and the regulator MITF⁷. On the contrary, high concentrations of CBD have been also described to inhibit tyrosinase activity and melanin synthesis⁸. Therefore, although some results may indicate some conditions or concentrations where the effect of CBD provides inhibition of melanin production, CBD is mostly described to increase melanin production in the state of the art.

Pine bark extracts contain proanthocyanidins that are among the most abundant compounds in various pine tree species. It has been shown that a polymeric proanthocyanidin fraction of Pine bark induces a significant increase in melanogenesis. However, polyphenolic fractions from Pine bark significantly inhibited tyrosinase activity⁹. In addition, Pine bark extract combined with b-arbutin or sodium ascorbate also showed whitening activity in melanoma cells¹⁰.

*Coriandrum sativum extract,* known as coriander, is a plant extract that can also affect melanogenesis. In one study a Coriander extract showed 47,76% of tyrosinase inhibitory activity¹¹, and in another study showed 13.56% of tyrosinase inhibitory activity¹².

Nowadays, there are other underlying factors involved in hyperpigmented skin that are not present in many cosmetic preparations focused on des-pigmentation. Because of this, there is a need for a formulation to treat hyperpigmented skin not only by inhibiting melanin production, but also facing the effects of photo-aging through the inhibition of ROS and the induction of melanin degradation in melanosomes by an autophagic process.

### Brief description of the invention

Present invention refers to a composition comprising Cannabidiol, *Coriandrum sativum* extract, and *Pinus radiata* bark extract.

One aspect of the invention refers to a non-therapeutic use of the above referred composition or the above referred cosmetic formulation, for aesthetically reducing and/or preventing the appearance of skin hyperpigmentation.

Present invention also refers to a non-therapeutic cosmetic method for reducing melanin production comprising identifying a portion of the skin in need of melanin reduction; and topically applying to said portion of the skin the composition of the invention.

Additionally, present invention refers to a cosmetic formulation comprising the above referred composition and further comprising at least one cosmetically acceptable excipient or vehicle.

A second aspect of present invention refers to a pharmaceutical formulation comprising a therapeutic effective amount of the above referred composition and further comprising at least one pharmaceutically acceptable excipient or vehicle.

Yet another aspect of present invention refers to the above referred composition, or to the above referred pharmaceutical formulation, for use as a medicament, in particular for use in the prevention and/or treatment of a dermatological hyperpigmentation disorder.

### Brief description of the figures

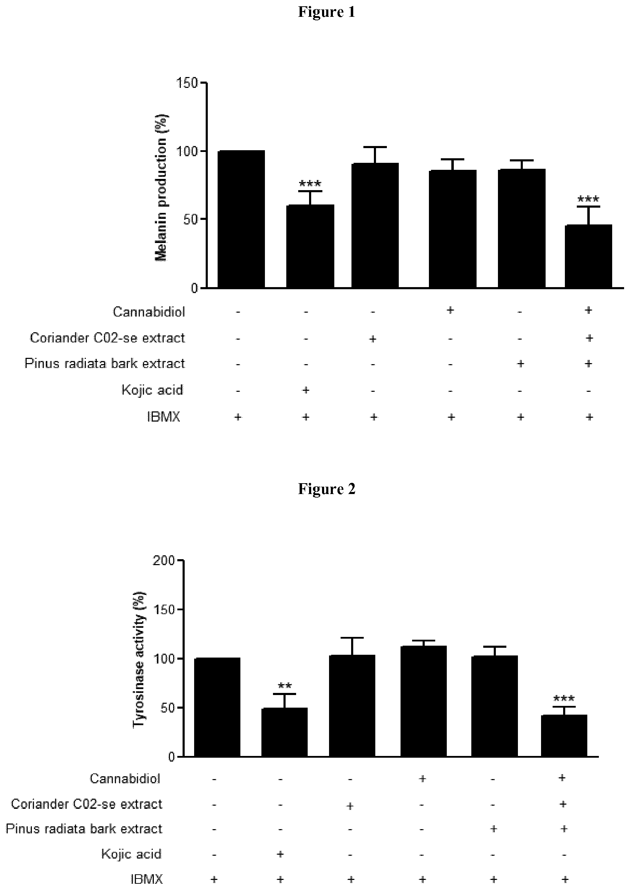

**Figure 1****: Effects of the composition of the invention (INH-002) on melanin production.** To analyze the effect on melanogenesis, mouse B16 melanoma cells were treated with the compounds (cannabidiol, *Coriandrum sativum* extract and *Pinus radiata* bark extract, as well as the composition of the invention) at the indicated doses for 72 hours. 3-Isobutyl-1 -methylxanthine (IBMX) 0.1 mM was used as melanogenesis inductor and Kojic acid (2 mM) as a positive control of bleaching. Melanin production was obtained by measurements of absorbance at 405 nm using a TECAN Genios Pro (Tecan Group Ltd.). Data are the means of three independent experiments ± SD. ***P<0.001 compared with IBMX group.
**Figure 2****: Effects of the composition of the invention (INH-002) on tyrosinase activity.** B16 melanoma cells were incubated with the compounds (cannabidiol, *Coriandrum sativum* extract and *Pinus radiata* bark extract, as well as the composition of the invention) at the indicated doses for 72 hours. IBMX (0.1 mM) and Kojic acid (2 mM) were used as inductor and inhibitor of tyrosinase activity, respectively. The tyrosinase activity was evaluated by monitoring the DOPAchrome through the measurement of absorbance at a wavelength of 475 nm in a microplate reader (TECAN Genios Pro, Tecan Group Ltd.). Data are the means of three independent experiments ± SD. ***P<0.001, **P<0.01 compared with IBMX group.

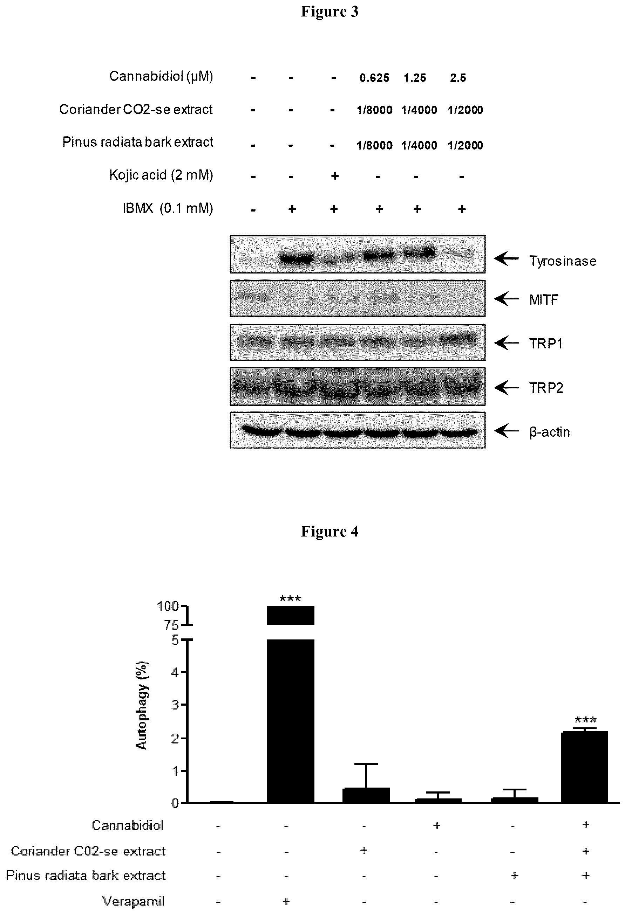

**Figure 3****: Effect of the composition of the invention (INH-002) on melanogenesis-related protein expression.** B16 melanoma cells were cultured with increasing concentrations of INH-002 combination for 24 hours. Tyrosinase, MITF, TRP1, and TRP2 proteins levels were analyzed by western blotting by using specific antibodies.
**Figure 4****: Effects of the composition of the invention (INH-002) on autophagy activity.** HaCaT cells were seeded in deprived hormones medium and incubated for 48 h with the compounds (cannabidiol, *Coriandrum sativum* extract and *Pinus radiata* bark extract, as well as the composition of the invention) at the indicated doses. Verapamil (10 µM) was used as a positive control. Autophagy activity was determined with fluorescence probe cyto-ID using IncuCyte ZOOM Live-Cell Imaging System. ***P<0.001, *P<0.05 compared with untreated cells.

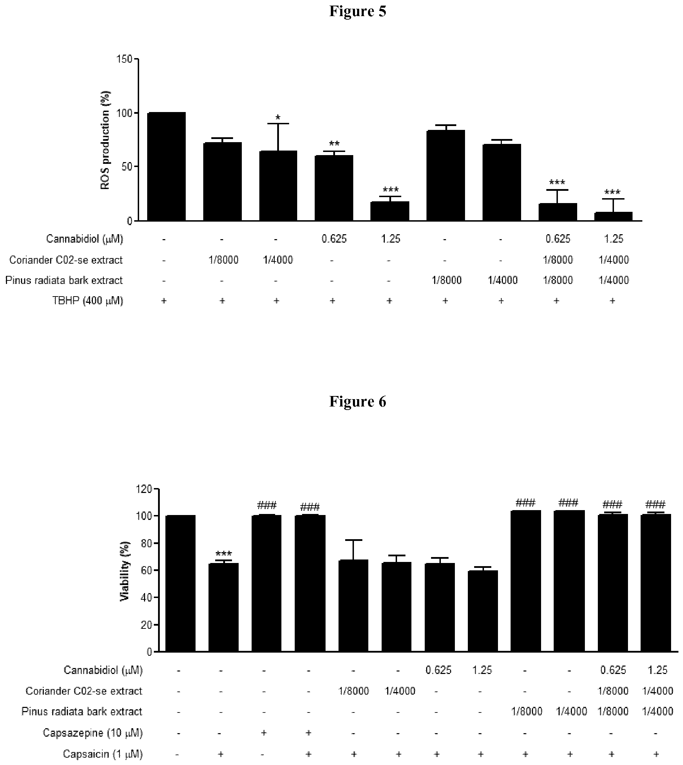

**Figure 5****: Effects of the composition of the invention (INH-002) on ROS production in HaCaT cells.** HaCaT cells were incubated with the compounds (cannabidiol, *Coriandrum sativum* extract and *Pinus radiata* bark extract, as well as the composition of the invention) at the indicated doses. TBHP (400 µM) is used as ROS inductor. ***P<0.001, **P<0.01, *P<0.05 compared with TBHP group.
**Figure 6****: Effects of the composition of the invention (INH-002) on TRPV1 channels in HEK-293T-TPRV1 cells.** HEK-293T-TPRV1 cells were pre-incubated for 3 h with the compounds (cannabidiol, *Coriandrum sativum* extract and *Pinus radiata* bark extract, as well as the composition of the invention) at the indicated doses. Capsaicin (1 µM) and capsazepine (10 µM) are used as agonist and antagonist controls, respectively. ***P<0.001 compared with untreated cells. ### P<0.001 compared with the capsaicin group.

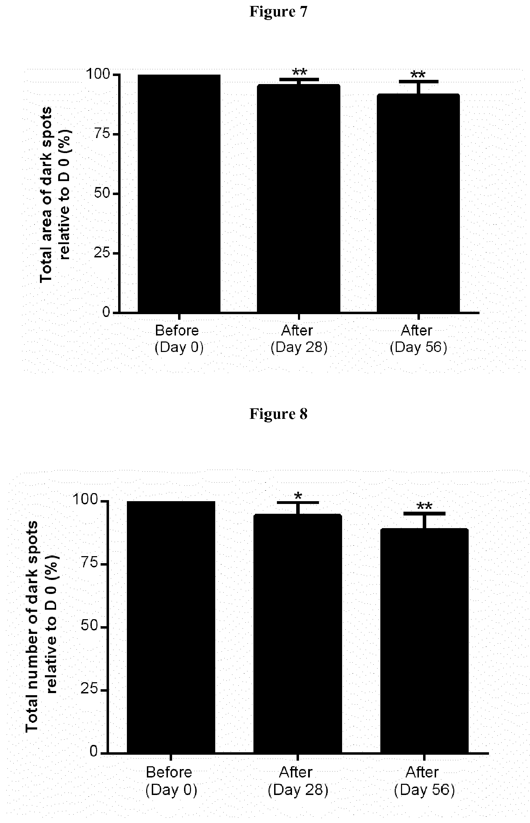

**Figure 7****: Effect of the composition of the invention (INH-002) on the total area of dark spots (*in vivo*).** Total area of dark spots in the region of interest, before treatment, after 1 month of treatment and after 2 months of treatment with the composition of the invention, in 20 human volunteers, comparing each volunteer with its own control before the treatment. **P< 0.05 compared with the day before the treatment.
**Figure 8****: Effect of the composition of the invention (INH-002) on the total number of dark spots (*in vivo*).** Total number of dark spots recognized by software in a region of interest, before treatment, after 1 month of treatment and after 2 months of treatment with the composition of the invention, in 20 human volunteers, comparing each volunteer with its own control before the treatment. **P< 0.05 compared with the day before the treatment.

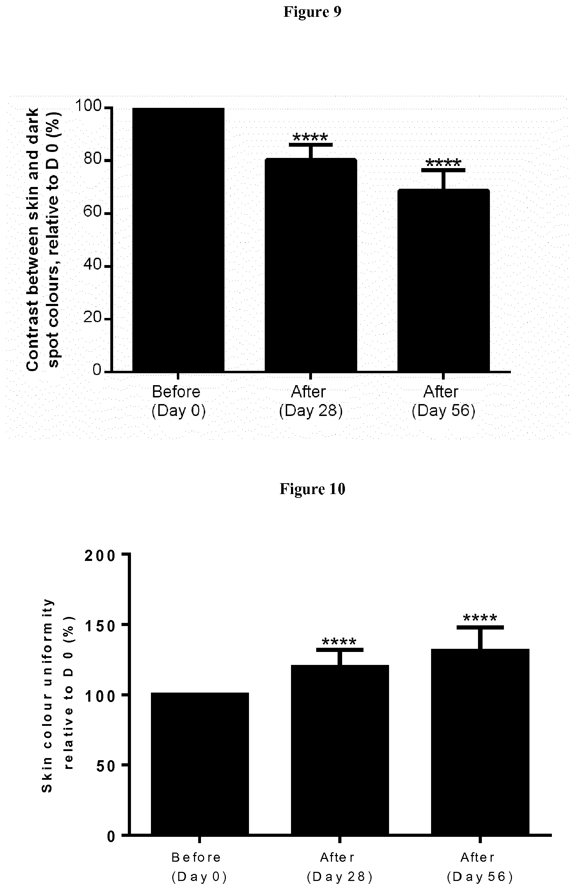

**Figure 9****: Effect of the composition of the invention (INH-002) on the contrast between skin and dark spot colors (*in vivo*).** Graphical representation of the skin color contrast between normal skin and a dark spot area in a region of interest, before treatment, after 1 month of treatment and after 2 months of treatment with the INH-002 composition, in 20 human volunteers, comparing each volunteer with its own control before the treatment. ****P<0.0001 compared with the day before the treatment.
**Figure 10****: Effect of the composition of the invention (INH-002) on skin color uniformity (*in vivo*).** Graphical representation of skin color uniformity for normal skin and a dark spot area in the region of interest, before treatment, after 1 month of treatment and after 2 months of treatment with the INH-002 composition, in 20 human volunteers, comparing each volunteer with its own control before the treatment. ****P<0.0001 compared with the day before the treatment.

### Detailed description of the invention

Present invention refers to a composition comprising Cannabidiol, *Coriandrum sativum* extract, and *Pinus radiata* bark extract. In one embodiment the composition of the invention comprises the same % (v/v) of *Coriandrum sativum* extract and *Pinus radiata* bark extract. In another embodiment the composition of the invention comprises a ratio of *Coriandrum sativum* extract in % (v/v) to cannabidiol in % (w/v) of 0.6 and a ratio of *Pinus radiata* bark extract in % (v/v) to cannabidiol in % (w/v) of 0.6.

In particular, said composition has the capability of inhibiting melanin production via the inhibition of the tyrosinase activity as well as increasing the degradation of melanin by inducing autophagic activity as shown in the examples of present application.

This composition has the capability of reducing the production of melanin, via inhibition of the tyrosinase activity, and through the activation of autophagy which increases melanin degradation. Said capability makes the composition of the invention useful for cosmetic purposes by aesthetically reducing and/or preventing the appearance of skin hyperpigmentation, as well as for therapeutic purposes for the prevention and/or treatment of a dermatological hyperpigmentation disorder.

"Hyperpigmentation" as used herein, refers to an area of skin wherein the pigmentation is greater than that of an adjacent area of skin (e.g., a pigment spot, an age spot, and the like).

Surprisingly, it has been discovered that cannabidiol, *Coriandrum sativum* extract and *Pinus radiata* bark extract act synergistically to inhibit melanin synthesis. Without being limited to theory, it is believed that this inhibition of melanin synthesis may lead to reduced melanin levels in the upper layers of the epidermis

Thus, the composition of the invention surprisingly provides synergic activity at different cellular and molecular pathways involved in melanogenesis, as shown in the examples of present application, wherein the combination of ingredients, present in the composition of the invention, provided synergistic results according to the Chou-Talalay algorithm (Cancer Res. 2010; 70: 440-446). This method provides the theoretical basis for the combination index (CI)-isobologram equation that allows quantitative determination of drug interactions, where CI < 1, = 1, and > 1 indicate synergism, additive effect, and antagonism, respectively. On the contrary, this effect is not observed when evaluating the components separately.

As shown in figure 1 and example 1, using IBMX (3-isobutyl-1-methylxanthine) as melanogenesis inductor and kojic acid as a positive control of bleaching (whitening), the composition of the invention significantly inhibited melanin production in B16 melanoma cell cultures, whereas each of the components at the same concentration did not.

Figure 2 and figure 4 show the mechanisms responsible for the synergetic reduction of melanin production observed for the composition of the invention.

In particular, figure 2 and example 2, show how the tyrosinase activity, a key enzyme in melanogenesis pathway, decreases synergistically when the composition of the invention is applied in B16 melanoma cell cultures, whereas said effect is not achieved when each of the components at the same concentration is applied separately.

Additionally, at protein expression level, the composition of the invention reduces the amount of tyrosinase and MITF in B16 melanoma cell cultures, when compared with the IBMX treatment (example 3 and figure 3).

Moreover, figure 4 (example 4) shows the ability of the composition of the invention to synergistically induce autophagic activity *in vitro* in human keratinocytes cell cultures. In pigmented skin, autophagic activity is lower than in lighted skin, whereas said effect is not achieved when each of the components at the same concentration is applied separately. The composition of the invention synergistically promotes, therefore, the autophagic activity of keratinocytes and melanin degradation.

On the other hand, in melanogenesis, the effect of UV-radiation contributes to oxidative stress induction. UV radiation produces reactive oxygen species (ROS) in the skin that may induce melanogenesis by increasing tyrosinase activity, due to its preference for the superoxide anion radical over oxygen. In this sense, the composition of the invention provides an important antioxidant activity, significantly inhibiting the reactive oxygen species (ROS) production *in vitro* in human keratinocytes cell cultures (example 5 and figure 5). This is particularly useful to ameliorate the signs of skin aging or photo-aging.

Importantly, the composition of the invention has also shown antagonist activity on the TRPV-1 channels (example 6 and figure 6) increasing significantly the viability of HEK-293T-TPRV1 cells. TPRV-1 (Transient receptor potential cation channel subfamily V member 1), also known as the capsaicin receptor, is related to the detection and regulation of body temperatures, as well as to the notion of pain (nociception). In this sense, the antagonist activity on the TRPV-1 channels of the composition of the invention, facilitates its topical use in sensitive and atopic skin.

In this sense, as mentioned previously, the composition of the invention, as described above herein, may be used, as a cosmetic composition, in non-therapeutic cosmetic uses and methods, aesthetically reducing and/or preventing the appearance of skin hyperpigmentation (e.g. dark spots, color imperfections, etc.), as well as reducing the aesthetic effects of photo-aging in the skin; but also as a pharmaceutical composition in therapeutic uses, in the prevention and/or treatment of dermatological hyperpigmentation disorders (e.g. melasma, lentigo, melanoma, etc.). Accordingly, the composition of the invention may be formulated in both cosmetic products (cosmetic formulations), or in pharmaceutical products (pharmaceutical formulations).

A first aspect of present invention refers, therefore, to a non-therapeutic use of the composition of the invention, as described above herein, as a cosmetic composition, for aesthetically reducing and/or preventing the appearance of skin hyperpigmentation. Preferably, for aesthetically whitening of the skin or for aesthetically lightening of the skin and reducing the aesthetic effects of photo-aging in the skin. In a preferred embodiment said composition is administered topically to the skin.

An embodiment of present invention refers, as well, to a non-therapeutic use of the composition of the invention, as a cosmetic composition, for aesthetically reducing and/or preventing the appearance of skin dark spots and/or freckles. Another embodiment of present invention refers to a non-therapeutic use of the composition of the invention, as a cosmetic composition, for aesthetically increasing skin color uniformity.

Another embodiment of the invention refers to a non-therapeutic cosmetic method for reducing and/or preventing the appearance of skin hyperpigmentation comprising administering the composition of the invention, as described above herein. Preferably, said method comprises administering the composition of the invention, as described above herein, at an amount effective to inhibit the production of melanin.

Yet another embodiment of present invention refers to a non-therapeutic cosmetic method for reducing melanin production. The cosmetic method comprises identifying a portion of the skin in need of melanin reduction; and topically applying to said portion of the skin the composition of the invention. Preferably, the composition is applied to the skin, in said cosmetic method, in a cosmetically effective amount.

For the purposes of present invention, a cosmetically effective amount of the composition of the invention, referred to the non-therapeutic cosmetic use thereof, refers to an amount sufficient to induce one or more aesthetical effects. Non-limiting examples of aesthetical effects include reducing and/or preventing the appearance of skin hyperpigmentation, whitening of the skin, lightening of the skin, improving skin color irregularities, ameliorate the signs of skin aging or photo-aging, reducing and/or preventing the appearance of skin dark spots and/or freckles, increasing skin color uniformity and/or improving the skin condition.

An embodiment of present invention refers, as well, to a non-therapeutic use of the composition of the invention for aesthetically reducing and/or preventing the appearance of skin dark spots and/or freckles. Another embodiment of present invention refers to a non-therapeutic use of the composition of the invention for aesthetically increasing skin color uniformity or for improving skin color irregularities.

Another embodiment of present invention refers to a cosmetic formulation comprising the composition of the invention, as described above herein, and, optionally, comprising at least one cosmetically acceptable excipient or vehicle.

In relation to this cosmetic use, methods and formulations, an *in vivo* study was carried out to test the activity of the composition of the invention (example 7). The topical use of the composition of the invention significantly showed whitening and anti-dark spots effects by reducing total area, total number and contrast of the dark spots, as well as increased skin color uniformity, after two months of application. The volunteer's opinion about cosmetic attributes and cosmetic efficacy were positively assessed using some parameters like skin illumination, unifies skin tone, etc.

Cosmetic formulations including cannabidiol, *Coriandrum sativum* extract and *Pinus radiata* bark extract may be used, for example, to improve skin color irregularities (e.g. hyperpigmentation), lighten the color of the skin, ameliorate the signs of aging, and/or improve skin condition.

The cosmetic formulations according to the invention may be in the form of emulsions (milk or cream), hydroalcoholic lotions, oily or oleo-alcoholic gels, dispersions or solid sticks, sprays or aerosol foams. The preferred compositions are lotions, milks or emollient creams, milks or creams and skin care foundation bases.

The vehicle may be aqueous, anhydrous or an emulsion. Preferably, the formulations are aqueous or an emulsion, especially water-in-oil or oil-in-water emulsions.

Among the cosmetically acceptable excipient or vehicles (carriers) which may be present in the cosmetic formulations according to the invention are fatty substances such as oils or mineral waxes, animal or vegetable fatty acids, fatty acid esters such as triglycerides of fatty acids having from 6 to 18 carbon atoms, fatty alcohols; emulsifying agents such as ethoxylated fatty alcohols or polyglycerol alkyl ethers; solvents such as lower monoalcohols or polyalcohols containing 1 to 6 carbon atoms, organic solvents or water.

Mono- or polyalcohols more particularly preferred are chosen from ethanol, isopropanol, propylene glycol, glycerol and sorbitol.

Examples of fats and mineral oils used include Vaseline oil; examples of animal oils used include whale oil, seal, menhaden, halibut liver, cod, tuna, turtle, ox foot, horse foot, sheep foot, mink, otter, marmot, etc .; examples of vegetable oils used include, almond oil, wheat germ, olive, corn germ, jojoba, sesame, sunflower, palm nuts, shea shorea, macadamia, blackcurrant seed and the like.

Examples of fatty acid esters used include acid esters C₁₂-C₂₂ saturated or unsaturated lower alcohols such as isopropanol or glycerol or fatty alcohols of C₈-C₂₂, linear or branched, saturated or unsaturated alkanediols or 1,2-C₁₀-C₂₂. Additional examples of fats are petrolatum, paraffin, lanolin, hydrogenated lanolin, tallow, acetylated lanolin and silicone oils.

Examples of waxes used include Sipol wax, lanolin wax, beeswax, candelilla wax, microcrystalline wax, carnauba wax, spermaceti, cocoa butter, shea butter, silicone waxes, hydrogenated oils solid at 25 ° C, sugar glycerides, oleates, myristates, linoleates and stearates of Ca, Mg and Al.

Examples of fatty alcohols used include lauryl, cetyl, myristyl, stearyl, palmityl and oleyl alcohols Guerbet such as 2-octyldodecanol, 2-decyltetradecanol or 2-hexyl decanol.

Thickeners may also be utilized as part of the cosmetically acceptable carrier of compositions according to the present invention. Examples of thickeners include crosslinked acrylates (e.g. Carbopol 982), hydrophobically-modified acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for use in the present invention include guar, xanthan, sclerotium, carrageenan, pectin and combinations thereof.

An oil or oily material may be present, together with an emulsifier, to provide either a water-in-oil emulsion or an oil-in-water emulsion, depending largely on the average hydrophilic-lipophilic balance (HLB) of the emulsifier employed.

Surfactants may also be present in cosmetic compositions of the present invention. The surfactant may be selected from the group consisting of anionic, nonionic, cationic and amphoteric actives. Particularly preferred nonionic surfactants include C₁₀-C₂₀ fatty alcohols or acids condensed with 2 to 100 moles of ethylene oxide or propylene oxide per mole; C2-C10 alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soaps, alkyl ether sulfate and sulfonates, alkyl sulfates and sulfonates, alkylbenzene sulfonates, alkyl and dialkyl sulfosuccinates, C8-C20 acyl isethionates, acyl glutamates, C8-C20 alkyl ether phosphates and combinations thereof.

In a preferred embodiment, the cosmetic formulation, as described above herein is administered topically. In another preferred embodiment the cosmetic formulation, as described above herein is a skin care product.

Additionally, in a preferred embodiment said cosmetic formulations comprise at least one additional cosmetically active ingredient. The term "cosmetically active ingredient" refers to a compound, extract or substance that when applied to the skin, provides a cosmetic benefit or improvement to the skin.

In this sense, the cosmetic formulations may include other known skin care compounds and still provide a synergistic effect. In an embodiment said cosmetically active ingredient is a skin whitening substance or compound selected from the group consisting of hydroquinone, resorcinol, arbutin, kojic acid, azelaic acid, vitamin C, glutathione, niacinamide, aleosin, resveratrol, glabidrin, soyabean, retinoic acid, octadecenedioic acid, glycolic, lactic acid, *Broussonetica kazwoki* extract, *B. Papyfera* fruit extract, *Cornus officinalis* root extract, *Rhus javanica* extract, *Pinus densiflora* extract, grape seed extract, orchid extract, Aloe vera extract, pycnogenol, Marine algae extract, cinnamic acid, flavonoids, green tea extract, coffeeberry, mulberry extract, licorice extract, umbelliferone, coswellia, retinoids, or other compounds which reduce melanin synthesis or increase melanin degradation.

The cosmetic formulations may also optionally contain one or more UV protective substance. As used herein, "UV protective substance" includes both sunscreen agents and physical sunblocks. Suitable UV protective substances may be organic or inorganic.

In another embodiment said additional cosmetically active ingredient is a compound for improving the firmness of the skin, improving the hydration status or moisturization of the skin, improving the appearance of fine lines and/or wrinkles, improving skin exfoliation or desquamation, improving skin barrier properties, improving skin tone, and/or improving the brightness or translucency of skin etc.

One embodiment refers to a non-therapeutic use of the cosmetic formulation, comprising the composition of the invention as described above herein, for aesthetically reducing and/or preventing the appearance of skin hyperpigmentation. Preferably, for aesthetically whitening of the skin or for aesthetically lightening of the skin and reducing the aesthetic effects of photo-aging in the skin. In a preferred embodiment said composition is administered topically to the skin.

An embodiment of present invention refers, as well, to a non-therapeutic use of the cosmetic formulation, comprising the composition of the invention as described above herein, for aesthetically reducing and/or preventing the appearance of skin dark spots and/or freckles. Another embodiment of present invention refers to a non-therapeutic use of the cosmetic formulation as described above herein, for aesthetically increasing skin color uniformity.

Another embodiment of the invention refers to a non-therapeutic cosmetic method for reducing and/or preventing the appearance of skin hyperpigmentation comprising administering the cosmetic formulation, comprising the composition of the invention as described above herein. Preferably, said method comprises administering the cosmetic formulation as described above herein, at an amount effective to inhibit the production of melanin.

Yet another embodiment of present invention refers to a non-therapeutic cosmetic method for reducing melanin production. The cosmetic method comprises identifying a portion of the skin in need of melanin reduction; and topically applying to said portion of the skin the cosmetic formulation, comprising the composition of the invention as described above herein. Preferably, the composition is applied to the skin, in said cosmetic method, in a cosmetically effective amount.

A further embodiment of present invention refers, as well, to a non-therapeutic use of the cosmetic formulation, comprising the composition of the invention as described above herein, for aesthetically reducing and/or preventing the appearance of skin dark spots and/or freckles. Another embodiment of present invention refers to a non-therapeutic use of the cosmetic formulation, comprising the composition of the invention as described above herein, for aesthetically increasing skin color uniformity or for improving skin color irregularities.

A second aspect of the invention refers to the therapeutic use of a composition, as described above herein, in therapy, in particular in the prevention and/or treatment of dermatological hyperpigmentation disorders.

Skin disorders, such as acne, atopic dermatitis or lichen planus, may be accompanied by localized post-inflammatory hyperpigmentation. Patients suffering from primary adrenal insufficiency (Addison's disease) also suffer from hyperpigmented skin.

In this sense, an embodiment refers to the composition of the invention, as described above herein, for use as a medicament.

Another embodiment refers to the composition of the invention, as described above herein, for use in the prevention and/or treatment of a dermatological hyperpigmentation disorder. In an embodiment, said hyperpigmentation disorder is melasma (or chloasma), lentigo, melanoma, hyperpigmentation produced by the use of a medicament, post-inflammatory hyperpigmentation or hyperpigmentation derived from Addison's disease.

Another embodiment of the invention refers to a pharmaceutical formulation comprising a pharmaceutically effective amount of the composition of the invention, as described above herein, and optionally comprising at least one pharmaceutically acceptable excipient or vehicle, an optionally at least one pharmaceutically acceptable excipient or vehicle.

For the purposes of present invention, a pharmaceutically effective amount of the composition of the invention, in relation to the therapeutic pharmaceutical uses thereof, refers to an amount effective to inhibit the production of melanin. However, said amount varies depending on the condition being treated, as well as, for example the age, weight, and clinical condition of the patient. Other factors include: the route of administration, the patient, the patient's medical history, the severity of the disease process, and the potency of the particular compound. The amount should be sufficient to ameliorate symptoms without producing unacceptable toxicity to the patient. In general, an effective amount of the composition is that which provides either subjective relief of symptoms or an objectively identifiable improvement as noted by the clinician or other qualified observer.

The pharmaceutical formulation, described above herein, may include at least one pharmaceutically acceptable excipient, which may be a carrier or a diluent, by a way of example. In making said pharmaceutical formulation, conventional techniques for the preparation of pharmaceutical formulations may be used. For example, the composition of the invention will usually be mixed with a carrier, or diluted by a carrier, or enclosed within a carrier. When the carrier serves as a diluent, it may be solid, semi-solid, or liquid material that acts as a vehicle, excipient, or medium for the active composition. The composition can be adsorbed on a granular solid container. Some examples of suitable carriers are water, salt solutions, alcohols, polyethylene glycols, polyhydroxy ethoxylated castor oil, peanut oil, olive oil, lactose, terra alba, sucrose, cyclodextrin, amylose, magnesium stearate, talc, gelatine, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxyl methylcellulose, and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations may be formulated so as to provide quick, sustained, or delayed release of the active ingredient after administration to the patient by employing procedures well known in the art.

The pharmaceutical formulations can be sterilized and mixed, if desired, with auxiliary agents, emulsifiers, salt for influencing osmotic pressure, buffers and/or colouring substances and the like, which do not deleteriously react with the active composition.

To prepare topical formulations, the composition is placed in a dermatological vehicle as is known in the art. The amount of the composition to be administered and the concentration in the topical formulations depend upon the vehicle, delivery system or device selected, the clinical condition of the patient, the side effects and the stability of the compound in the formulation. Thus, the physician employs the appropriate preparation containing the appropriate concentration of the composition and selects the amount of formulation administered, depending upon clinical experience with the patient in question or with similar patients.

Another embodiment refers, therefore, to a pharmaceutical formulation comprising a pharmaceutically effective amount of the composition of the invention, as described above herein, for use as a medicament.

A preferred embodiment of the invention refers to a pharmaceutical formulation comprising a pharmaceutically effective amount of the composition of the invention, as described above herein, for use in the prevention and/or treatment of a dermatological hyperpigmentation disorder.

Another embodiment refers to a method of preventing and/or treating a dermatological hyperpigmentation disorder, said method comprising administering to a subject in need thereof, a pharmaceutically effective amount of the pharmaceutical formulation as described above herein.

### Examples

### Example 1: Effect of the composition of the invention on melanin production.

The composition of the invention comprising Cannabidiol, *Coriandrum sativum* extract, and *Pinus radiata* bark extract was evaluated. For the preparation of the composition were used:
- Cannabidiol with >90% purity
- Phytoextract *Coriandrum sativum* contains 75 - 97 % essential oil, 48 - 80 % volatile compounds (linalool, alpha-pinene, limonene, linalyl acetate, gamma-terpinene, camphor, aldehydes).
- Phytoextract *Pinus radiata* bark extract contains 4-8% *Pinus radiata* bark extract, 40-50% aqua, and 40-50% glycerin.

The skin hyperpigmentation is caused by a melanin production alteration. With the aim of investigating the role of the composition of the invention in melanin production, a cellular assay system was used to evaluate the *in vitro* depigmenting activity, in B16 melanoma cell cultures co-treated with IBMX, a melanogenesis inductor.

B16 melanoma cell line was maintained in supplemented DMEM medium with 10% FBS and 1% antibiotics penicillin/streptomycin at 37°C in a humidified atmosphere of 5% CO₂. The day before the assay, cells were seeded at a density of 5 x 10⁴ cells/well in 24 well-plate with 1 ml of DMEM and incubated for 24 h at 37°C. Then 24 h later, cells were pre-treated with the composition of the invention and the individual ingredients of the combination for 30 min. Then wells pre-treated with compounds were treated with IBMX 0.1 mM. Kojic acid 2 mM, an inhibitor of tyrosinase, was used as negative control. 72 h later, cells were washed with phosphate buffer saline (PBS) and raised by trypsinization. Then, cells were collected with DMEM in 1.5 ml tubes and centrifugated at 1300 rpm for 2 min. The float was discarded, and the pellet was washed with PBS and centrifugated again. The pellet was dissolved in 100 µl of NaOH 1M solution and incubated at 60°C for 30 minutes in thermoblock. Finally, the production of melanin was determined by absorbance measurements at 405 nm in TECAN Genious Pro. The 100% melanin production is given to positive control (cells treated with IBMX 0.1 mM). The results represent the mean and standard deviation of three independent experiments.

The melanin inhibition by the composition of the invention was considered if the inhibition was equal or major than half inhibition of kojic acid versus the control. The treatment of B16 melanoma cells with composition of the invention exhibited a significant melanin inhibition (P < 0.001) compared with IBMX group (figure 1). Moreover, this combination shows a synergic activity determinate by the Chou and Talalay method (Combination Index CI = 0.91).

### Example 2: Effect of the composition of the invention on tyrosinase activity.

Tyrosinase is an essential enzyme in the melanin production pathway. In order to determinate the inhibitory tyrosinase activity of the composition of the invention, a biological assay in B16 melanoma cell line was performed.

This melanoma cell line was maintained in supplemented DMEM medium with 10% FBS and 1% antibiotics penicillin/streptomycin at 37°C in a humidified atmosphere of 5% CO₂. These cells were seeded at a density of 5 x 10⁴ cells/well in 24-well plates with 1ml of DMEM and incubated at 37°C for 24 h. The day after, cell cultures were pre-treated with the composition of the invention and the individual ingredients of the combination for 30 min. Then, wells pre-treated with compounds were treated with IBMX 0.1 mM. Kojic acid 2 mM, an inhibitor of tyrosinase, was used as negative control. Then of 72 h, cells were washed with PBS and raised by trypsinization. After, cells are collected with DMEM in 1.5 ml tubes and centrifugated at 1300 rpm for 2 min. The float was removed, and the pellet was washed with PBS and centrifugate again. Then, 30 µl the lysis buffer (proteases inhibitors) was added to each tube and incubated on ice for 30 min. After the lysate was centrifugate at 13000 rpm for 2 min, the float obtained is the source of tyrosinase and was put it in 96-well plate. Then, it was added 70µl/well of L-DOPA 0.07% in buffer phosphate 50 mM pH 6.8. The plate was incubated at 37°C for 2 h. Finally, DOPAchrome formation is monitored by measuring absorbance at 450nm in TECAN Genious Pro.

The results represent the mean and standard deviation of three independent experiments.

The 100% tyrosinase activation corresponds to positive control (cells treated with IBMX 0.1 mM). The tyrosinase inhibition by each component of the composition, or by the composition of the invention, was considered if the inhibition was equal or major than the half inhibition of kojic acid versus the control. The treatment of B16 melanoma cells with the composition of the invention exhibited a significant tyrosinase inhibition (P < 0.001) compared with IBMX group. This inhibition was even greater than the inhibition produced by kojic acid (figure 2).

The synergic activity was also determined by the Chou and Talalay method. Table 1, shows the combination index obtained (Chou-Talalay) at two different concentrations, but using the same ratio of the ingredients, in relation to the capability thereof to reduce tyrosinase activity:

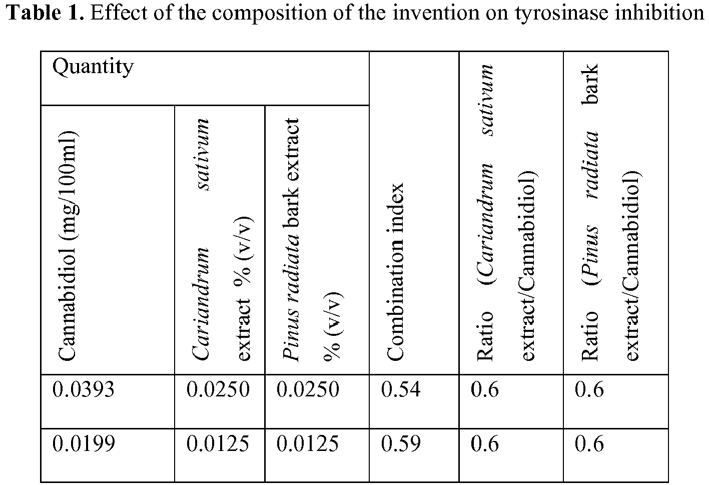

### Example 3: Effect of the composition of the invention on melanogenesis-related protein expression

In the melanogenesis pathway, tyrosinase, MITF, TRP1, and TRP2 are key proteins. The reduction of their levels produces a decrease of the melanin production and thus, of the skin pigmentation. To determinate the role of the composition of the invention in protein-related expression melanogenesis, tyrosinase, MITF, TRP1, and TRP2 proteins levels were analyzed by western blot.

Whole cell extracts were obtained by lysing B16 melanoma cells in NP-40 buffer (50 mM Tris-HCl pH 7.5, 150 mM NaCl, 10% glycerol and 1% NP-40) supplemented with protease and phosphatase inhibitors. Lysate concentrations were determined by the Bradford assay (Bio-Rad Laboratories, Hercules, CA, USA). Proteins (50 µg/lane) were separated by SDS-PAGE, transferred onto PVDF membranes and blocked with PBS-T (Phosphate-buffered saline and 0.1% Tween-20) containing 5% non-fat dry milk for 1 h at room temperature (RT). Incubation with primary antibodies was performed overnight at 4°C, followed by incubation with the appropriate horseradish peroxidase-conjugated secondary antibody. The washed membranes were incubated with appropriate secondary antibodies coupled to horseradish peroxidase that were detected by an enhanced chemiluminescent reagent.

Figure 3 confirms that the treatment with the composition of the invention reduced tyrosinase and MITF protein expression levels in B16 melanoma cells compared with cells treated with IBMX.

### Example 4: Effect of the composition of the invention on autophagy activity.

Autophagy has a pivotal role in skin color determination because it regulates melanosome degradation in keratinocytes, and thereby contributes to the diversity of skin color. In fact, keratinocytes derived from caucasian skin exhibit higher autophagic activity than those derived from african skin. In this context, a cellular assay was performed to determinate the autophagic activity of human keratinocyte treated with the composition of the invention.

HaCaT cell line was maintained in supplemented RPMI medium with 10% FBS and 1% antibiotics penicillin/streptomycin at 37°C in a humidified atmosphere of 5% CO₂. The day before the assay, cells were seeded at a density of 75 x 10³ cells/well in 96-well black plate with RPMI and incubated at 37°C for 24 h. The next day, cell cultures were treated with the composition of the invention and the individual ingredients of the combination or Verapamil 10 µM (autophagy inductor) in Earle's Balanced Salt Solution medium (deprived hormones medium). 48 h later, the medium was removed and washed two times with IX assay buffer to each well. Then, the buffer was removed and added 100 µl/well of CYTO-ID Green Detection Reagent (1µl/ml) into the culture medium without Phenol Red indicator. The plate was incubated at 37°C for 30 min. Then, cells were washed twice with 100 µl/well IX assay buffer. Finally, was added 100µl/well assay buffer and introduced the plate into IncuCyte ZOOM live cell microscopy. The images were taken, and the fluorescence analyzed using the IncuCyte FLR software. The results represent the mean and standard deviation of three independent experiments.

The result obtained with verapamil is considered as 100% autophagy activity. The treatment with the composition of the invention induced significantly autophagy activity (P < 0.001) in HaCaT cells compared with control (figure 4). The ability of the composition of the invention to induce autophagic activity improves the capability to use it in cosmetic and pharmaceutical formulations with skin whitening effects.

The synergic activity was also determined by the Chou and Talalay method. Table 2, shows the combination index obtained (Chou-Talalay) at two different concentrations, but using the same ratio of the ingredients, in relation to the capability thereof to activate autophagy:

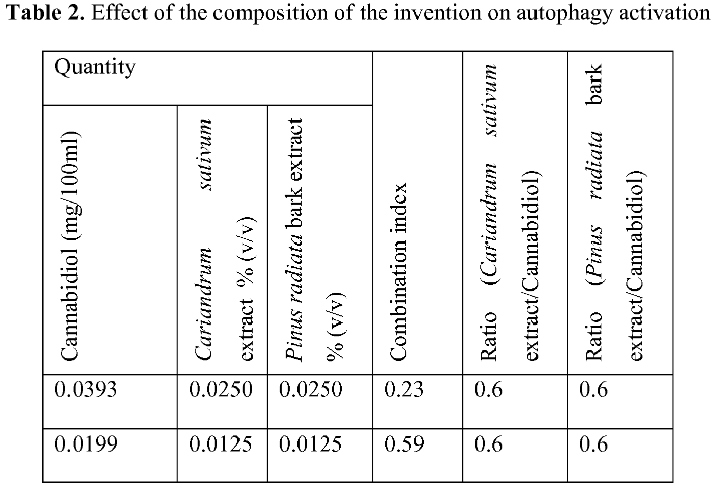

### Example 5: Effect of the composition of the invention on ROS production.

In melanogenesis, the effect of UV-radiation contributes to oxidative stress induction. UV radiation produces reactive oxygen species (ROS) in the skin that may induce melanogenesis by increasing tyrosinase activity, due to its preference for the superoxide anion radical over oxygen. For this reason, a cellular assay was performed to determinate the ROS inhibitory activity of the composition of the invention in a human keratinocyte cell culture.

HaCaT cell line was maintained in supplemented RPMI medium with 10% FBS and 1% antibiotics penicillin/streptomycin at 37°C in a humidified atmosphere of 5% CO₂. Cells were seeded at a density of 1 x 10⁴ cells/well in 96-well plate with RPMI and incubated at 37°C for 24 h. The next day, cell cultures were pre-treated with the composition of the invention and the individual ingredients of the combination for 30 min. Then, cells pre-treated with compounds were treated with TBHP 400 µM and incubated at 37° in darkness for 3 hours. Below, the culture medium was removed, and it was added 100 µl/well of CM-H₂DCFDA 1µM in PBS for 20 min at 37°C. The medium was removed, and cells washed twice with PBS. Finally, the plate was introduced into IncuCyte ZOOM and the fluorescence was measured. The results represent the mean and standard deviation of three independent experiments.

The 100% of ROS production is given to the TBHP treatment. Both doses of the composition of the invention significantly inhibited the ROS production (P < 0.001) when compared with TBHP (figure 5). The composition of the invention is a powerful antioxidant beneficial for the hyperpigmented skin.

### Example 6: Effect of the composition of the invention on TRPV-1 channels

TRPV-1 is an ionotropic channel the activation of which by a specific agonist such as capsaicin mobilizes intracellular calcium in TRPV-1 expressing cells. Capsaicin-induced cytotoxicity is a model of TRPV-1 agonistic assay in HEK-293T-TRPV1 cells. In an antagonism assay, cells are treated with ingredients in combination with capsazepine, an antagonist of TRPV-1. The antagonist effect of the composition of the invention in TRPV-1 channels was performed by biological assay.

The HEK-293T-TRPV1cell line was maintained in supplemented DMEM with 10% FBS, 1% antibiotics penicillin/streptomycin and 400 µg/ml of geneticin at 37°C in a humidified atmosphere of 5% CO₂. The day before the assay, cells were seeded at a density of 1 x 10⁴ cells/well in 96-well plate and incubate at 37°C for 24 h. The next day, cells were pre-treated with the composition of the invention and the individual ingredients of the combination in RPMI with YOYO-1 iodide 0.1 µM for 30 min. Capsazepine 10 µM was used as antagonist agent. Then, cells pre-treated with compounds were treated with Capsaicin 1 µM for 3 h. YOYO-1 is a cell impermeant cyanine dimer nucleic acid stain that can only enter cells with a compromised plasma membrane and fluorescently stain the nuclear DNA. Later, YOYO-1 fluorescence was measured using the IncuCyte ZOOM. Finally, endpoint analysis was performed to measure the total number of DNA containing objects by treating cells with 0.0625% triton X-100 to permeabilize the cell membrane.

The 100% of TRPV-1 activation corresponds to untreated cells. The treatment with the composition of the invention inhibited significantly the TRPV-1 channel activation (P < 0.001) compared with capsaicin (figure 6). Although TRPV-1 antagonism is not a pathway involved in melanogenesis, the ability of the composition of the invention to inhibit TRPV-1 channels allows their application in sensitive and atopic skins.

### Example 7: In vivo whitening efficacy assessment of the composition of the invention

Once that all the *in vitro* tests showed that the composition of the invention has a significant and synergic depigmenting activity, the next step was carrying out an *in vivo* clinical study.

For the efficacy test, 20 volunteers were submitted to two months of treatment with the composition of the invention in the whole facial area, twice a day. All of them showed hyperpigmented facial areas.

Images of each volunteer were done with Bio Blue Light Scanner from the part of the face with more presence of dark spots. The pictures were taken before the treatment, after 28 days of treatment and after 56 days of treatment. Blue Light Scanner is able to analyze skin pigmentation by dividing the region of interest in hypopigmented and hyperpigmented areas, in order to assess the efficacy of cosmetic treatments.

Then, images were processed through specific software, the region of interest (ROI) was selected and 3 parameters (total area of dark spots, the total number of dark spots, and the contrast between normal skin and dark spots) were obtained for each of the measurements. Additionally, each volunteer answered a self-assessment questionnaire at the end of the treatment. All data were statistically analyzed applying Paired Student's t-test, since each volunteer shows a pair of values (before and after the treatment).

The results show that treatment with the composition of the invention for two months significantly reduced the total area (figure 7), the total number (figure 8) and the contrast (figure 9) of the dark spots by 8.5 ± 2.8%, 11.2 ± 3.0% and 31.3 ± 3.7%, respectively, whereas increased skin color uniformity was increased by 31.3 ± 3.7% (figure 10), comparing each volunteer with its own control before the treatment (paired Student's t-test).

Additionally, the volunteers answered a self-assessment questionnaire where the *in vivo* results were confirmed through significant positive evaluation. For a significant percentage of volunteers, it was considered that the application of the product was easy, quickly absorbed and with a pleasant texture, perfume, and color.

### References

1. Serre C, Busuttil V, Botto J-M. Intrinsic and extrinsic regulation of human skin melanogenesis and pigmentation. Int J Cosmet Sci. 2018;40(4):328-347. doi:10.1111/ics.12466
2. D'Mello SAN, Finlay GJ, Baguley BC, Askarian-Amiri ME. Signaling pathways in melanogenesis. Int J Mol Sci. 2016;17(7):1-18. doi:10.3390/ijms17071144
3. Gillbro JM, Olsson MJ. The melanogenesis and mechanisms of skin-lightening agents - Existing and new approaches. Int J Cosmet Sci. 2011;33(3):210-221. doi: 10.1111/j.1468-2494.2010.00616.x
4. Denat L, Kadekaro AL, Marrot L, Leachman SA, Abdel-Malek ZA. Melanocytes as instigators and victims of oxidative stress. J Invest Dermatol. 2014;134(6):1512-1518. doi:10.1038/jid.2014.65
5. Sarkar R, Arora P, Garg KV. Cosmeceuticals for Hyperpigmentation: What is Available? J Cutan Aesthet Surg. 2013;6(1):4-11. doi:10.4103/0974-2077.110089
6. Hwang YS, Kim Y-J, Kim MO, et al. Cannabidiol upregulates melanogenesis through CB1 dependent pathway by activating p38 MAPK and p42/44 MAPK. Chem Biol Interact. 2017;273:107-114. doi:10.1016/J.CBI.2017.06.005
7. Pucci M, Pasquariello N, Battista N, et al. Endocannabinoids stimulate human melanogenesis via type-1 cannabinoid receptor. J Biol Chem. 2012;287(19): 15466-15478. doi:10.1074/jbc.M111.314880
8. ZHANG KE; TAN XIN; YU ZHAOHUI; CHEN ZHENG; WU YANAN; LI MENG. Applications of cannabidiol or cannabis extract in preparing skin whitening products. 2018:20.
9. Ku CS, Mun SP. Effect of Pinus radiate bark extracts with different molecular weight distributions on cell growth of NIH/3T3 fibroblasts and dendrite retraction of B16 melanoma cells. J Wood Sci. 2011;57(5):415-420. doi:10.1007/s10086-011-1193-y
10. YASUMURO MISAO; SATO MANAMI. Oral cavity composition. 2005:15.
11. Mukherjee PK, Badami S, Wahile AM, Rajan S, Suresh B. Evaluation of Tyrosinase Inhibitory Activity of some Indian Spices. J Nat Remedies. 2001;1(2):125-129. doi:10.18311/JNR/2001/20
12. Kamkaen N, Kamkaen N, Mulsri N, Treesak C. Original Article Screening of Some Tropical Vegetables for Anti-tyrosinase Activity. http://citeseerx.ist.psu.edu/viewdoc/summary?doi=10.1.1.606.6926. Accessed September 12, 2018.

## Claims

1. A composition comprising Cannabidiol, *Coriandrum sativum* extract, and *Pinus radiata* bark extract.

2. A cosmetic formulation comprising the composition according to claim 1, and optionally at least one cosmetically acceptable excipient or vehicle.

3. A cosmetic formulation according to claim 2, further comprising at least one additional cosmetically active ingredient.

4. A non-therapeutic use of a composition according to claim 1, or of a cosmetic formulation according to any of claims 2 or 3, for aesthetically reducing and/or preventing the appearance of skin hyperpigmentation and photoaging.

5. A non-therapeutic use according to claim 4, for aesthetically whitening of the skin.

6. A non-therapeutic use according to any of claims 4 or 5, for aesthetically reducing and/or preventing the appearance of skin dark spots and/or freckles.

7. A non-therapeutic cosmetic method for reducing and/or preventing the appearance of skin hyperpigmentation comprising administering the composition according to claim 1.

8. A non-therapeutic cosmetic method according to claim 7, wherein the composition according to claim 1 is administered topically.

9. A non-therapeutic cosmetic method for reducing melanin production comprising identifying a portion of the skin in need of melanin reduction; and topically applying to said portion of the skin the composition of the invention.

10. A composition according to claim 1, for use as a medicament.

11. A composition according to claim 1, for use in the prevention and/or treatment of a dermatological hyperpigmentation disorder.

12. A pharmaceutical formulation comprising a pharmaceutically effective amount of the composition according to claim 1, and optionally at least one pharmaceutically acceptable excipient or vehicle.

13. A pharmaceutical formulation according to claim 12, further comprising at least one additional pharmaceutically active ingredient.

14. A pharmaceutical formulation according to any of claims 12 or 13, for use as a medicament.

15. A pharmaceutical formulation according to any of claims 12 or 13, for use in the prevention and/or treatment of a dermatological hyperpigmentation disorder.
